**Europäisches Patentamt**

(19) **European Patent Office**

**Office européen des brevets**

(11) Veröffentlichungsnummer: **0 046 534**
**B1**

(12) **EUROPÄISCHE PATENTSCHRIFT**

(45) Veröffentlichungstag der Patentschrift:
**11.07.84**

(21) Anmeldenummer: **81106225.6**

(22) Anmeldetag: **10.08.81**

(51) Int. Cl.³: **C 07 C 37/74,** C 07 C 37/60,
C 07 C 39/08, C 07 C 39/04

(54) Verfahren zur Herstellung von Brenzcatechin und Hydrochinon.

(30) Priorität: **22.08.80 DE 3031736**

(43) Veröffentlichungstag der Anmeldung:
**03.03.82 Patentblatt 82/9**

(45) Bekanntmachung des Hinweises auf die Patenterteilung:
**11.07.84 Patentblatt 84/28**

(84) Benannte Vertragsstaaten:
**AT BE CH DE FR GB IT LI NL**

(56) Entgegenhaltungen:
**DE - A - 1 518 317**
**FR - A - 2 146 195**
**FR - A - 2 322 120**
**FR - A - 2 375 174**

**INDUSTRIAL & ENGINEERING CHEMISTRY, Product Research and Development Band 15, Nr. 3, 1976, Seiten 212-215 Washington, D.C., U.S.A. J. VARAGNAT: "Hydroquinone and pyrocatechol production by direct oxidation of phenol**
**CHEMICAL ABSTRACTS, Band 89, Nr.19, 6. November 1978, Seite 545, Nr. 163250h Columbus, Ohio, U.S.A.**

(73) Patentinhaber: **BAYER AG, Konzernverwaltung RP Patentabteilung, D-5090 Leverkusen 1 Bayerwerk (DE)**

(72) Erfinder: **Jupe, Christoph, Dr.,**
**Franz-Peter-Kürten-Weg 2, D-5000 Köln 80 (DE)**
Erfinder: **Waldmann, Helmut, Dr.,**
**Henry-T.-von-Böttinger-Strasse 15, D-5090 Leverkusen (DE)**
Erfinder: **Baumert, Jürgen, An den Kreutzmorgen 7, D-5000 Köln 80 (DE)**
Erfinder: **Schümmer, Günther, Weissdornweg 1, D-5024 Stommeln (DE)**

## Beschreibung

Die vorliegende Erfindung betrifft ein Verfahren zur Herstellung von Brenzcatechin und Hydrochinon.

Brenzcatechin und Hydrochinon sind technisch wichtige organische Feinchemikalien, die sowohl direkt, z.B. in photographischen Entwicklern, als auch als Zwischenprodukte für z.B. Farbstoffe, Polymerisationsinhibitoren, Pharmazeutika und Pflanzenschutzmittel Verwendung finden (s. Kirk-Othmer, „Encyclopedia of Chemical Technology", 2. Aufl., Bd. 11, S. 462 bis 492, insbesondere S. 469 und 488 [1966]).

Die Suche nach wirtschaftlichen und einfachen Herstellungsverfahren führte u.a. zu einer Reihe von Phenoloxidationsverfahren, die Brenzcatechin und Hydrochinon als Koppelprodukte liefern (s. z.B. DE-OS Nrn. 2658943, 2410742, 2364181, 2658545, 2332747, 1593968, 2633302, 2064497, 2150657, 2167040, 2341743, 2407398, 1543953, 2404114 sowie japanische Patentanmeldungen Nrn. 54-55530 und 54-66629 und T. Tsuchiya, M. Andoh und J. Imamura, „Nipp. Kag. Kaishi", 1979, 3, S. 370 bis 374).

Bei diesen Verfahren wird Phenol mit einem peroxidischen Reagenz umgesetzt, z.B. mit Wasserstoffperoxid oder einer Percarbonsäure, die meist in einem niedrigerer als Phenol siedenden Lösungsmittel gelöst sind. Ein weiteres Charakteristikum dieser Verfahren besteht darin, dass zur Vermeidung von Überoxidation ein Unterschuss an Oxidationsmittel, bezogen auf die zu hydroxylierende Verbindung, eingesetzt wird. Dies hat zur Folge, dass nach der Reaktion nicht umgesetztes Phenol im Reaktionsgemisch enthalten ist.

Besonders günstige Bedingungen ergeben sich, wenn man die Hydroxylierung mit Percarbonsäuren durchführt, da hierbei z.B. die Umsetzungen ohne Zusätze von Katalysatoren durchgeführt werden können (s. beispielsweise DE-OS Nr. 2658943).

In der bisher erwähnten Literatur wird zur Aufarbeitung der nach der Hydroxylierung vorliegenden Reaktionsgemische meist auf übliche Methoden verwiesen, vor allem auf Destillation, Extraktion und Kristallisation. Genauere Angaben fehlen weitgehend.

Ausführliche Angaben zur Reaktion und Aufarbeitung findet man in zwei Veröffentlichungen, die technische Anlagen zur Herstellung von Brenzcatechin und Hydrochinon aus Phenol und Wasserstoffperoxid beschreiben, und zwar bei Jean Varagnat, „Ind. Eng. Chem., Prod. Res. Dev.", Bd. 15, Nr. 3, S. 212 bis 215 (1976) und P. Maggioni und F. Minisci, „La Chimica e l'Industria", Bd. 59, Nr. 4, S. 239 bis 242 (1977).

In der Veröffentlichung von J. Varagnat wird dargelegt, wie in einer Folge von fünf Rektifikationskolonnen das Reaktionsgemisch destillativ getrennt wird. Phenol und weitere Hilfs- und Begleitstoffe werden in vier Kolonnen als Kopfprodukte gewonnen und wiederverwendet, während Brenzcatechin und Hydrochinon in einer fünften Kolonne getrennt werden und das Hydrochinon anschliessend einer Kristallisation unterworfen wird.

Auch gemäss dem von P. Maggioni und F. Minisci beschriebenen Verfahren wird das Reaktionsgemisch hauptsächlich destillativ aufgearbeitet. Nach einer stufenweisen Verdampfung in drei hintereinander geschalteten getrennten Verdampfern bei jeweils niedrigerem Druck bis hinunter zu 13 mbar erfolgt die restliche Auftrennung des Gemisches in zwei Rektifikationskolonnen. In der ersten Kolonne wird Phenol zur Rückführung und in der zweiten Kolonne werden die Produkte Brenzcatechin und Hydrochinon erhalten.

Betrachtet man den Aufwand, der für die technische Aufarbeitung von Reaktionsgemischen aus Phenol und peroxidischen Reagenzien zur Gewinnung von Brenzcatechin und Hydrochinon getrieben wird, so kann man feststellen, dass bei den beiden zuvor beschriebenen Verfahren vor allem die Abtrennung des nicht umgesetzten Phenols einen grossen Aufwand erfordert.

Es wurde nun ein Verfahren zur Herstellung von Brenzcatechin und Hydrochinon durch Umsetzung von Phenol mit Percarbonsäuren mit 1 bis 4 Kohlenstoffatomen bei einem Molverhältnis von Phenol zu Percarbonsäure vor der Reaktion von 5:1 bis 50:1 und Aufarbeitung des nach der Reaktion und ggf. nach weiterer Behandlung vorliegenden Gemisches, das nicht umgesetztes Phenol, die der Percarbonsäure entsprechende Carbonsäure, Brenzcatechin, Hydrochinon und ggf. weitere Bestandteile enthält, unter Verwendung kontinuierlich betriebener Rektifikationsapparate gefunden, das dadurch gekennzeichnet ist, dass man

a) das Gemisch kontinuierlich einer ersten Rektifikationskolonne, die bis zu 20 Trennstufen im Abtriebs- und 5 bis 30 Trennstufen im Verstärkungsteil aufweist, zwischen Abtrieb- und Verstärkungsteil oder dem Sumpfbereich zuführt, diese Kolonne bei einem Druck zwischen 0,01 und 2 bar betreibt, zwischen 20 und 95 Gew.-% des Kopfproduktes oder die gleiche Gewichtsmenge an Phenol oder an einem Phenol enthaltenden Produktstrom aus dem Verfahren oder die gleiche Gewichtsmenge eines Gemisches aus Kopfprodukt und Phenol und/oder einem Phenol enthaltenden Produktstrom aus dem Verfahren kondensiert als flüssigen Rücklauf oben auf die Kolonne zurückführt, ein Kopfprodukt abzieht, das Phenol und Carbonsäure und ggf. weitere Bestandteile, die tiefer sieden als Phenol, enthält, und ein Sumpfprodukt entnimmt, das Brenzcatechin, Hydrochinon, 40 bis 65 Gew.-% Phenol und ggf. weitere Bestandteile enthält, und aus dem Sumpfprodukt Brenzcatechin und Hydrochinon gewinnt, und

b) das Kopfprodukt der ersten Rektifikationskolonne kontinuierlich einer zweiten Rektifikationskolonne, die 5 bis 35 Trennstufen im Verstärkungs- und 8 bis 35 Trennstufen im Abtriebsteil aufweist, zwischen Abtriebs- und Verstärkungsteil zuführt, diese Kolonne bei einem Druck zwischen 0,02 und 2 bar betreibt, 20 bis 95 Gew.-% des Kopfproduktanfalles kondensiert als flüssigen Rücklauf oben auf die Kolonne zurückführt, ein Kopfprodukt abzieht, das praktisch phenolfrei ist und das die der Percarbonsäure entsprechende Carbonsäure enthält und dem Abtriebsteil und/oder dem Sumpf der Kolonne ein praktisch reines Phenol entnimmt.

Ein in die erfindungsgemässe destillative Aufarbeitung einsetzbares Gemisch kann nach bekannten Verfahren erhalten werden, indem man Phenol mit einer Percarbonsäure mit 1 bis 4 Kohlenstoffatomen bei einem Molverhältnis von Phenol zu Percarbonsäure vor der Reaktion von 5:1 bis 50:1 umsetzt und das dabei erhaltene Reaktionsgemisch ggf. weiter behandelt.

Als Percanbonsäure können Monopercarbonsäuren eingesetzt werden, die sich von Monocarbonsäuren mit gleicher Anzahl von Kohlenstoffatomen ableiten, z.B. Peressigsäure oder Perpropionsäure. Es ist möglich, nur eine Percarbonsäure für sich alleine einzusetzen. Man kann aber auch ein Gemisch von mehreren Percarbonsäuren mit 1 bis 4 Kohlenstoffatomen einsetzen. Bevorzugt ist es, eine Percarbonsäure mit 2, 3 oder 4 Kohlenstoffatomen jeweils für sich alleine einzusetzen. Besonders bevorzugt ist es, Peressigsäure oder Perpropionsäure jeweils für sich alleine einzusetzen.

Die Umsetzung von Phenol mit Percarbonsäure wird im allgemeinen bis zu einem weitgehenden Umsatz der Percarbonsäure durchgeführt. Bevorzugt ist ein Umsatz an Percarbonsäure von über 99%, besonders bevorzugt von mehr als 99,7%, so dass das in die erfindungsgemässe destillative Aufarbeitung einzusetzende Gemisch weitgehend percarbonsäurefrei ist.

Ggf. kann das aus der Umsetzung von Phenol und Percarbonsäure erhaltene Gemisch vor dem Einsatz in die erfindungsgemässe destillative Aufarbeitung einer weiteren Behandlung unterzogen werden. Diese kann beispielsweise darin bestehen, dass man Phosphorsäure in einem Reaktionsgemisch aus Phenol und Persäure vor der Destillation neutralisiert (s. DE-OS Nr. 2364181). Eine andere Art der weiteren Behandlung kann z.B. darin bestehen, dass man von dem flüssigen Reaktionsgemisch eine Gasphase abzieht, die z.B. Sauerstoff und Kohlendioxid als Nebenprodukte der Hydroxylierungsreaktion enthalten kann.

Die in die erfindungsgemässe destillative Aufarbeitung einzusetzenden Gemische enthalten Phenol, die der Percarbonsäure entsprechende Carbonsäure, Brenzcatechin, Hydrochinon und ggf. weitere Bestandteile.

Der Gehalt an Phenol kann in weiten Grenzen schwanken. Im allgemeinen liegt er zwischen 5 und 95, vorzugsweise zwischen 20 und 90 Gew.-%. Es ist jedoch auch möglich, Gemische mit davon abweichenden Phenolgehalten in die erfindungsgemässe destillative Aufarbeitung einzusetzen.

Zur wirtschaftlichen Durchführung der erfindungsgemässen destillativen Aufarbeitung ist das Gewichtsverhältnis von Phenol zu Brenzcatechin und Hydrochinon im einzusetzenden Gemisch von Bedeutung. Im allgemeinen ist es vorteilhaft, in die erfindungsgemässe destillative Aufarbeitung Gemische einzusetzen, bei denen dieses Gewichtsverhältnis möglichst gering ist. Aufgrund der Tatsache, dass die Hydroxylierung von Phenol vorteilhafterweise in Gegenwart eines grossen Phenolüberschusses durchgeführt wird, liegen jedoch im allgemeinen in den Reaktionsgemischen nach der Hydroxylierung erhebliche Mengen unumgesetzten Phenols vor. Solche Reaktionsgemische, in denen das Gewichtsverhältnis von Phenol zu Brenzcatechin und Hydrochinon beispielsweise im Bereich 3,4 bis 100:1 oder 6 bis 40:1 liegt, sind zum Einsatz in die erfindungsgemässe destillative Aufarbeitung geeignet. Die ggf. durchgeführte weitere Behandlung des Reaktionsgemisches aus der Hydroxylierung, z.B. die Neutralisation vorhandener Phosphorsäure oder die Abtrennung einer Gasphase, wird im allgemeinen so durchgeführt, dass das Mengenverhältnis von Phenol zu Brenzcatechin und Hydrochinon in den zuvor genannten Grenzen bleibt.

Es ist bevorzugt, das nach der Reaktion vorliegende Verhältnis von Phenol zu Brenzcatechin und Hydrochinon nicht durch eine ggf. durchgeführte weitere Behandlung zu verändern. Vorzugsweise wird also das nicht umgesetzte Phenol nach der Reaktion weitgehend durch die erfindungsgemässe destillative Aufarbeitung entfernt.

Besonders geeignet zum Einsatz in die erfindungsgemässe destillative Aufarbeitung sind Reaktionsgemische, bei denen das Molverhältnis von Phenol zu Percarbonsäure zwischen 8 und 25:1 im Einsatz in die Reaktion gelegen hat.

Der Gehalt an Carbonsäure im einzusetzenden Gemisch kann ebenfalls in weiten Grenzen schwanken. Er kann z.B. zwischen 1 und 95 Gew.-% oder zwischen 5 und 40 Gew.-% liegen.

Der Gehalt an Brenzcatechin und Hydrochinon im einzusetzenden Gemisch kann zwischen sehr kleinen Werten und etwa 21 Gew.-% schwanken. Obwohl ein möglichst grosser Gehalt an Brenzcatechin und Hydrochinon an sich bevorzugt ist, können wegen in der Regel grosser Anteile an nicht umgesetztem Phenol, nicht unerheblicher Anteile an Carbonsäure und wegen ggf. vorliegender sonstiger Bestandteile in vielen Fällen nur Gemische in das erfindungsgemässe Verfahren eingesetzt werden, die einen Gehalt von Brenzcatechin und Hydrochinon von unter 20 Gew.-% aufweisen. Häufig liegt der Gehalt an Dihydroxybenzolen im einzusetzenden Gemisch zwischen 0,5 und 10 Gew.-%.

Das Gewichtsverhältnis von Brenzcatechin zu Hydrochinon im Einsatzgemisch in die erfindungsgemässe destillative Aufarbeitung ist nicht von besonderer Bedeutung. Im allgemeinen liegt dieses Verhältnis zwischen 0,1 und 10 oder zwischen 0,8 und 4.

Die in die erfindungsgemässe destillative Aufarbeitung einzusetzenden Gemische können neben den bisher aufgeführten Bestandteilen ggf. weitere Bestandteile enthalten. Der Gehalt an weiteren Bestandteilen kann in weiten Grenzen schwanken. Er kann z.B. zwischen 0,1 und 50 Gew.-% liegen. Die möglicherweise vorhandenen weiteren Bestandteile können Siedepunkte unterhalb oder oberhalb des Siedepunktes von Phenol haben. Sie können auch sehr hochsiedend oder nicht destillierbar sein. Falls weitere Bestandteile ganz oder teilweise, ggf. im Gemisch mit anderen, bereits erwähnten Bestandteilen des einzusetzenden Gemisches, unter den Bedingungen der erfindungsgemässen destillativen Aufarbeitung phenolhaltige azeotrope Gemische bilden, ist ihr Anteil im einzusetzenden Gemisch so klein zu halten, dass aus dem einzusetzenden Gemisch nicht das gesamte Phenol azeotrop destilliert werden kann.

Bevorzugt sind Gemische, bei deren destillativer Aufarbeitung keine phenolhaltigen azeotropen Gemische als Kopfprodukte auftreten. Es ist ferner bevorzugt, Gemische in die erfindungsgemässe destillative Aufarbeitung einzusetzen, die, wenn sie weitere Bestandteile enthalten, nur solche weitere Bestandteile enthalten, deren Siedepunkte entweder niedriger als der Siedepunkt von Phenol oder höher als der Siedepunkt von Brenzcatechin liegen. Das bedeutet, es sind solche Gemische bevorzugt, in denen keine zwischen Phenol und Brenzcatechin siedenden Verbindungen enthalten sind.

Besonders bevorzugt sind Gemische, die, wenn sie überhaupt weitere Bestandteile enthalten, nur solche weitere Bestandteile enthalten, die entweder niedriger als die niedrigstsiedende, im Gemisch enthaltene Carbonsäure oder höher als Brenzcatechin sieden. Ganz besonders bevorzugt sind Gemische, die solche weitere Bestandteile enthalten, die tiefer als die niedrigstsiedende, im Gemisch vorhandene Carbonsäure sieden und die, falls sie noch andere weitere Bestandteile enthalten, nur noch solche andere weitere Bestandteile enthalten, die entweder oberhalb von Hydrochinon sieden oder unter den Bedingungen der erfindungsgemässen destillativen Aufarbeitung nicht destillierbar sind.

Die ggf. vorhandenen weiteren Bestandteile können auf verschiedenen Wegen in das einzusetzende Gemisch gelangt sein. Sie können chemisch von sehr unterschiedlicher, teilweise nicht einmal genau bestimmter Natur sein. Als Beispiele für weitere Bestandteile seien genannt:

— Lösungsmittel, z.B. aus einer zur Hydroxylierungsreaktion eingesetzten Percarbonsäurelösung, beispielsweise Wasser, Benzol, Chlorbenzol, 1,2-Dichlorpropan, 1,2-Dichlorethan, Essigsäureethylester, Essigsäure und/oder Propionsäure;
— Sauerstoff, der z.B. aus der Zersetzung eines peroxidischen Reagenzes stammen kann;
— Kohlenoxide (Kohlenmonoxid und/oder Kohlendioxid), die z.B. durch Überoxidation in der Hydroxylierungsreaktion entstanden sein können;
— Salze von Säuren, die z.B. bei der Neutralisation von Säuren im Reaktionsgemisch entstanden sein können;

— Trihydroxybenzole, die z.B. bei der Hydroxylierungsreaktion als Nebenprodukte entstanden sein können;
— höher als Hydrochinon siedende oder nicht destillierbare Substanzen, die sich z.B. ähnlich wie Braunkohle oder wie Huminsäuren oder teerartig verhalten und die z.B. als Nebenprodukte der Hydroxylierungsreaktion entstanden sein können;
— höher als Hydrochinon siedende oder nicht destillierbare, Phosphor enthaltende Substanzen oder andere, höher als Hydrochinon siedende Substanzen mit metallkomplexierenden Eigenschaften, die z.B. dem Hydroxylierungsgemisch zugesetzt worden sein können oder die sich aus zugesetzten Komplexbildnern gebildet haben können.

Die ggf. vorhandenen weiteren Bestandteile sind vorzugsweise so beschaffen, dass sie unter den Bedingungen der erfindungsgemässen destillativen Aufarbeitung nicht oder nur zu einem geringen Teil mit sich selber, untereinander oder mit anderen Bestandteilen des einzusetzenden Gemisches reagieren können.

Im allgemeinen werden höchstens 10 Gew.-%, besonders bevorzugt weniger als 5 Gew.-% irgendeiner Komponente des einzusetzenden Gemisches während der erfindungsgemässen destillativen Aufarbeitung durch Reaktion verbraucht.

Das vorbeschriebene Gemisch wird zur Durchführung der erfindungsgemässen destillativen Aufarbeitung kontinuierlich einer ersten Rektifikationskolonne zwischen Verstärkungs- und Abtriebsteil zugeführt.

Diese erste Rektifikationskolonne weist bis zu 20 Trennstufen im Abtriebsteil und 5 bis 30 Trennstufen im Verstärkungsteil auf. Für den Verstärkungsteil sind 5 bis 20 Trennstufen bevorzugt; besonders bevorzugt sind hierfür 7 bis 16 Trennstufen. Für den Abtriebsteil ist eine geringe Zahl von Trennstufen bevorzugt.

Es hat sich für Gemische, die hochsiedende oder nicht destillierbare organische Substanzen enthalten, die sich beispielsweise ähnlich wie Braunkohle oder wie Huminsäuren oder teerartig verhalten, als vorteilhaft erwiesen, solche Gemische dem Sumpfbereich der ersten Rektifikationskolonne zuzuführen. Eine solche Lage der Zulaufstelle ergibt einen Abtriebsteil mit der Wirkung von bis zu einer Trennstufe.

Eine Trennstufe ist hier und im folgenden definiert als ein Kolonnenabschnitt, dessen Trennwirkung bei einer Rektifikation einer einmaligen Gleichgewichtseinstellung zwischen aufsteigender Dampf- und absteigender Flüssigkeitsphase gleichkommt, wie es z.B. erläutert ist in „Organikum, Organisch-Chemisches Grundpraktikum", 15. Aufl. (Nachdruck), VEB Deutscher Verlag der Wissenschaften, Berlin, 1977, S. 63 bis 69, insbesondere S. 66 und 67.

Der sich an der Phasengrenze im Sumpf einstellende Zustand ist praktisch aus verschiedenen Gründen mehr oder weniger weit von einem idealen Gleichgewichtszustand entfernt, so dass dort die Trennwirkung unter der des Idealwertes von einer Trennstufe liegt.

Obwohl im Sumpfprodukt einer solchen Rektifikation noch geringe Mengen an leichtsiedenden Bestandteilen aus dem Zulaufgemisch vorhanden sein können, lässt sich das Ziel der erfindungsgemässen destillativen Aufarbeitung erreichen, nämlich eine Auftrennung des Einsatzgemisches unter Rückgewinnung von Phenol und ggf. weiteren Stoffen mit verringertem Aufwand.

Für Gemische, die weder hochsiedende noch nicht destillierbare Substanzen enthalten, ist es vorteilhaft, wenn die erste Rektifikationskolonne einen Abtriebsteil mit 6 bis 12 Trennstufen enthält.

Am Sumpf der ersten Rektifikationskolonne entnimmt man ein Gemisch, das 40 bis 65 Gew.-% Phenol, ggf. Spuren an leichter als Phenol siedenden Bestandteilen, Brenzcatechin, Hydrochinon sowie ggf. weitere Bestandteile, deren Siedepunkte höher als der Siedepunkt des Phenols liegen, enthält, und gewinnt daraus Brenzcatechin und Hydrochinon.

Das Sumpfprodukt enthält bevorzugt nicht mehr als 5 Gew.-% an Bestandteilen, deren Siedepunkte niedriger als der Siedepunkt des Phenols liegen; besonders bevorzugt ist ein Gehalt von weniger als 2,5 Gew.-% an solchen Leichtsiedern.

Beim erfindungsgemässen Betreiben der ersten Rektifikationskolonne gelingt es, Leichtsieder und einen grossen Teil des eingesetzten Phenols unter milden Bedingungen abzudestillieren. Die Sumpftemperatur kann dabei niedriger gehalten werden als in dem Falle, dass das gesamte Phenol bereits in dieser Stufe über Kopf entfernt werden müsste. Damit wird die thermische Belastung für das Sumpfprodukt gering gehalten. Diese Belastung ist hauptsächlich durch die Temperatur bedingt, bei der die zur Verdampfung nötige Wärmemenge im Verdampfer zugeführt wird und für die das Sumpfprodukt weitgehend als Wärmeüberträger dient. Ein weiterer Vorteil der erfindungsgemässen Arbeitsweise für die erste Rektifikationskolonne ist die Erniedrigung des Erstarrungspunktes des Sumpfproduktes, so dass der Aufwand gering ist, das Sumpfprodukt als Flüssigkeit zu handhaben. Es ist nämlich bevorzugt, das Sumpfprodukt der ersten Rektifikationskolonne als Flüssigkeit der nachfolgenden Gewinnung von Brenzcatechin und Hydrochinon zuzuführen.

Die Gewinnung von Brenzcatechin und Hydrochinon aus dem Sumpfprodukt der ersten Rektifikationskolonne kann in an sich bekannter Weise erfolgen, z.B. durch Destillation, Rektifikation, Kristallisation oder Extraktion oder eine beliebige Kombination mehrerer dieser Methoden, wobei man ggf. auch restliches Phenol und niedriger als Phenol siedende Bestandteile isolieren kann. Bevorzugt ist es, in weiteren Rektifikationskolonnen erst Leichtsiederreste und Phenol, dann Brenzcatechin und dann Hydrochinon als spezifikationsgerechte Destillate zu erhalten und abzuziehen. Ein diesem Weg entsprechendes Verfahren ist z.B. in der deutschen Patentanmeldung P Nr. 2928553.8 beschrieben. Es wurde gefunden, dass es keine Schwierigkeiten bereitet, nach dem in der vorgenannten deutschen Patentanmeldung beschriebenen Verfahren auch solche Gemische aufzuarbeiten, die ausser den dort erwähnten Bestandteilen noch einen gewissen Anteil an niedriger als Phenol siedenden Bestandteilen enthalten.

Die erste Rektifikationskolonne wird bei einem Druck zwischen 0,01 und 2 bar betrieben, jedoch unter Berücksichtigung der im folgenden erläuterten Temperaturgrenzen. Bevorzugt wird diese Kolonne zwischen 0,1 und 1,2 bar betrieben, ganz besonders bevorzugt zwischen 0,5 und 1,1 bar.

Die Temperaturen in der Kolonne stellen sich entsprechend dem Druck und der Zusammensetzung der Stoffgemische an den verschiedenen Stellen der Kolonne ein. Man betreibt die gesamte erfindungsgemässe destillative Aufarbeitung vorteilhaft so, dass produktseitig eine Temperatur von 250°C nicht überschritten wird. Bevorzugt ist es, produktseitige Temperaturen zwischen 230 und 250°C höchste kurzzeitig zu erreichen. Ganz besonders bevorzugt ist es, produktseitig stets Temperaturen unterhalb von 230°C einzuhalten. Die untere Grenze für Druck bzw. Temperatur ist durch den Erstarrungspunkt des Kopfproduktes gegeben. Die durch Druck und Zusammensetzung des Kopfproduktes bedingte Temperatur am Kopf der Kolonne muss oberhalb des Festpunktes des Kopfproduktes liegen, da sonst eine Rektifikation unter Aufgabe eines flüssigen, aus kondensiertem Kopfprodukt bestehenden Rücklaufes nicht durchgeführt werden kann.

Ggf. wird ein Teil des am Kopf der Kolonne anfallenden Dampfes kondensiert und als flüssiger Rücklauf auf die Kolonne zurückgegeben. Der nicht als Rücklauf auf die Kolonne zurückgegebene Teil des Kopfproduktes wird entnommen.

Das Mengenverhältnis von Rücklauf zu Entnahme liegt zwischen 0,25 und 19. Bevorzugt ist ein Rücklaufverhältnis zwischen 0,3 und 10, ganz besonders bevorzugt ein Rücklaufverhältnis zwischen 0,4 und 2,5.

Man kann anstelle von kondensiertem Kopfprodukt auch die gleiche Menge an flüssigem Phenol oder an einem Phenol enthaltenden flüssigen Produktstrom aus dem Verfahren als Rücklauf auf den Kopf der ersten Rektifikationskolonne geben. Es ist z.B. möglich, der zweiten Rektifikationskolonne der erfindungsgemässen destillativen Aufarbeitung im Abtriebsteil einen entsprechenden Produktstrom zu entnehmen. Bevorzugt kann ein solcher Produktstrom dem Sumpf der zweiten Rektifikationskolonne entnommen werden. Man kann auch Gemische von Kopfprodukt und flüssigem Phenol und/oder einem flüssiges Phenol enthaltenden Produktstrom aus dem Verfahren in der angegebenen Menge auf den Kopf der ersten Rektifikationskolonne geben.

Es ist bevorzugt, die Kopftemperatur der ersten Rektifikationskolonne so zu wählen, dass sie wenigstens 10 bis 15°C über der Sumpftemperatur der zweiten Rektifikationskolonne der erfindungsgemässen destillativen Aufarbeitung liegt, und die Dämpfe des Kopfproduktes der ersten Kolonne ganz oder teilweise als Heizmedium einem Wärmeaustauscher zuzuführen, der als Verdampfer für die zweite Rektifikationskolonne arbeitet, und die Dämpfe dort ganz oder teilweise zu kondensieren und das Kondensat ggf. weiter abzukühlen. Diese Kopplung der beiden Kolonnen gestattet es, für die Beheizung der Verdampfer mit einem Minimum an von aussen zuzuführender Energie auszukommen.

Zur besseren Ausnützung des Energieinhaltes der Dämpfe am Kopf der ersten Rektifikationskolonne kann man auch das Verfahren der sogenannten Brüdenkompression anwenden, und mit den auf ein höheres Druck- und Temperaturniveau gebrachten Brüden einen weiteren Verdampfer beheizen. Als weiterer Verdampfer kommt bevorzugt der Verdampfer der zweiten Rektifikationskolonne der erfindungsgemässen destillativen Aufarbeitung in Frage. Es ist aber auch möglich, einen anderen Verdampfer mit geeignetem Temperaturniveau auf diese Weise zu beheizen. Es kann ggf. weiterhin vorteilhaft sein, der ersten Rektifikationskolonne an geeigneter Stelle weitere Produktströme aus der erfindungsgemässen destillativen Aufarbeitung oder aus daran anschliessenden Aufarbeitungsstufen als Flüssigkeit oder Dampf oder als Flüssigkeits-/Dampf-Gemisch zuzuführen. Bevorzugt eignen sich dazu Produktströme, die in einer im Vergleich zum Einsatzgemisch in die erste Rektifikationskolonne kleinen Menge vorliegen und Phenol enthalten.

Ganz besonders bevorzugt führt man einen oder mehrere der folgenden Produktströme ganz oder teilweise zurück zur ersten Rektifikationskolonne:
— das restliche Leichtsieder und Phenol enthaltende Destillat, das man erhält, wenn man das Sumpfprodukt der ersten Rektifikationskolonne in einer weiteren Rektifikationskolonne von Restphenol und restlichen Leichtsiedern befreit;
— das Sumpfprodukt der zweiten Rektifikationskolonne der erfindungsgemässen destillativen Aufarbeitung, das überwiegend Phenol enthält, in dem Falle, dass man die Hauptmenge an Phenol oberhalb des Sumpfes der zweiten Rektifikationskolonne entnimmt;
— das Produkt, das man erhält, wenn man aus dem Kopfprodukt der zweiten Rektifikationskolonne die leichter als Phenol siedenden Bestandteile, z.B. durch Rektifikation, abgetrennt hat; das letztgenannte Produkt kann man aber auch z.B. der zweiten Rektifikationskolonne der erfindungsgemässen destillativen Aufarbeitung als zusätzlichen Zulauf zuführen.

Geeignet zur Zufuhr weiterer Produktströme sind die Stellen in der ersten Rektifikationskolonne, an denen die kolonneninternen Produktströme in ihrer Zusammensetzung den zusätzlich zuzuführenden Produktströmen möglichst ähnlich sind.

Das Kopfprodukt der ersten Rektifikationskolonne enthält Phenol, Carbonsäure sowie ggf. weitere Bestandteile, deren Siedepunkte niedriger liegen als der Siedepunkt von Phenol.

Es ist bevorzugt, so viel Phenol mit dem Kopfprodukt zu entnehmen, dass im Sumpfprodukt der ersten Rektifikationskolonne eine Phenolgehalt zwischen 30 und 70 Gew.-% vorliegt. Ganz besonders bevorzugt ist es, so viel Phenol mit dem Kopfprodukt der ersten Rektifikationskolonne zu entnehmen, dass im Sumpfprodukt ein Phenolgehalt zwischen 40 und 65 Gew.-% vorliegt.

Das am Kopf der ersten Rektifikationskolonne entnommene Produkt wird, ggf. nach der beschriebenen Ausnutzung seines Wärmeinhalts, einer zweiten Rektifikationskolonne kontinuierlich zwischen Verstärkungs- und Abtriebsteil zugeführt.

Diese zweite Rektifikationskolonne weist 5 bis 35 Trennstufen im Verstärkungs- und 8 bis 35 Trennstufen im Abtriebsteil auf. Bevorzugt ist eine Rektifikationskolonne mit 10 bis 30 Trennstufen im Abtriebs- und 10 bis 20 Trennstufen im Verstärkungsteil. Diese Kolonne wird bei einem Druck zwischen 0,02 und 2 bar, bevorzugt zwischen 0,05 und 1,2 bar und besonders bevorzugt zwischen 0,15 und 1,1 bar betrieben.

Das Kopfprodukt der zweiten Rektifikationskolonne wird teilweise kondensiert und in flüssiger Form als Rücklauf oben auf diese Rektifikationskolonne zurückgegeben. Der Anteil, der als Rücklauf auf die Kolonne zurückgegeben wird, liegt zwischen 20 und 95 Gew.-% des gesamten Kopfanfalles. Bevorzugt ist, 50 bis 90% des Kopfanfalles, besonders bevorzugt 60 bis 85 Gew.-% des Kopfanfalles als Rücklauf zu verwenden.

Das Kopfprodukt ist weitgehend phenolfrei und enthält die Carbonsäure. Bevorzugt ist ein Restphenolgehalt im Kopfprodukt von weniger als 1 Gew.-Teil Phenol auf 1000 Gew.-Teile Carbonsäure, ganz besonders bevorzugt ein Restphenolgehalt zwischen 10 und 500 Gew.-Teilen Phenol auf 1 Mio. Gew.-Teile Carbonsäure. Ggf. kann das Kopfprodukt der zweiten Rektifikationskolonne weitere Bestandteile enthalten, die niedriger als Phenol sieden.

Der nicht als Rücklauf verwendete Teil des Kopfproduktes wird entnommen und kann beliebig verwendet werden. Im allgemeinen wird man ihn kondensieren und in flüssiger Form weiter verwenden.

Bevorzugt ist es, die Carbonsäure aus dem Kopfprodukt der zweiten Rektifikationskolonne zu gewinnen und erneut zur Herstellung von Percarbonsäure zu verwenden, die ggf. für die Hydroxylierung von Phenol verwendet werden kann. Ebenso ist es bevorzugt, aus dem Kopfprodukt der zweiten Rektifikationskolonne ggf. enthaltenes Lösungsmittel zurückzugewinnen und erneut zu verwenden, z.B. zur Herstellung von Percarbonsäurelösungen, die ggf. für die Hydroxylierung von Phenol verwendet werden können. Die Gewinnung der Carbonsäure und ggf. des oder der Lösungsmittel kann in beliebiger Weise geschehen. Bevorzugt ist es, Carbonsäure und ggf. Lösungsmittel durch weitere Rektifikation in einer für eine erneute Verwendung ausreichend reinen Form zu gewinnen.

Aus dem Abtriebsteil der zweiten Rektifikationskolonne entnimmt man praktisch reines Phenol. Man kann das Phenol ausschliesslich als Sumpfprodukt abziehen, man kann aber auch einen Teil des Phenols als Flüssigkeit oder Dampf oder als Flüssigkeits-/Dampf-Gemisch zwischen Zulauf und Sumpf entnehmen. Bevorzugt ist es, den grössten Teil des aus dem Abtriebsteil zu entnehmenden Phenols als flüssigen Seitenstrom dem Abtriebsteil oberhalb des Kolonnensumpfes zu entnehmen. Ganz besonders bevorzugt ist es, den Phenolabzug aus dem Abtriebsteil der zweiten Rektifikationskolonne so aufzuteilen, dass zwischen 65 und 99,9 Gew.-% als Seitenstrom und entsprechend der Rest als Sumpfprodukt entnommen werden. Für die Entnahme eines Seitenstromes an Phenol ist im allgemeinen jede Stelle im Abtriebsteil zwischen 15 und 80% der gesamten wirksamen Länge des Abtriebsteils, vom Sumpf aus nach oben gezählt, geeignet.

Bevorzugt ist es, einen Seitenstrom an Phenol zwischen 20 und 60% der wirksamen Länge des Abtriebsteils, vom Sumpf aus nach oben gerechnet, zu entnehmen. Das aus dem Abtriebteil der zweiten Rektifikationskolonne entnommene Phenol kann Carbonsäure enthalten. Im allgemeinen liegt der Gehalt an Carbonsäure im entnommenen Phenol unter 5 Gew.-%, vorzugsweise unter 1 Gew.-%, ganz besonders bevorzugt zwischen 10 und 1500 Gew.-ppm.

Das entnommene Phenol kann einer beliebigen Verwendung zugeführt werden. Bevorzugt ist es, das Phenol zu kondensieren und in flüssiger Form ganz oder teilweise, ggf. nach weiterer Behandlung, erneut in die Umsetzung mit Percarbonsäure einzusetzen und einen ggf. nicht in die Umsetzung mit Percarbonsäure zurückgeführten Phenolanteil der ersten Rektifikationskolonne der erfindungsgemässen destillativen Aufarbeitung zuzuführen. Ganz besonders bevorzugt ist es, als Seitenstrom entnommenes Phenol flüssig, ggf. nach weiterer Behandlung, erneut in die Umsetzung mit Percarbonsäure einzusetzen.

Es kann weiterhin zweckmässig sein, einen phenolhaltigen Rückstand, wie er z.B. bei der weiteren Aufarbeitung des am Kopf der zweiten Rektifikationskolonne abgezogenen Produktes anfallen kann, der zweiten Rektifikationskolonne an geeigneter Stelle als zusätzlichen Zulauf zuzuführen. Es ist aber auch möglich, einen solchen Produktstrom der ersten Rektifikationskolonne als zusätzlichen Zulauf zuzuführen.

Hinsichtlich der Temperatureinschränkungen für die zweite Rektifikationskolonne gilt das für die erste Rektifikationskolonne Gesagte.

Für die Durchführung der erfindungsgemässen destillativen Aufarbeitung sind alle üblichen Rektifikationskolonnen geeignet, z.B. Füllkörper-, Siebboden- oder Glockenbodenkolonnen. Auch Kolonnen mit Gewebe- oder anderen Packungen sind gut geeignet. Es können auch Kolonnen verwendet werden, die in verschiedenen Abschnitten verschiedene Einbauten oder Füllkörper enthalten. Ebenso ist die Art der zu den Kolonnen gehörenden Verdampfer für die Durchführung der erfindungsgemässen destillativen Aufarbeitung nicht entscheidend, da alle gängigen Verdampfertypen eingesetzt werden können, z.B. Rohrbündelwärmeaustauscher oder Fallfilmverdampfer mit Zwangsumlauf.

Für die Fertigung der Rektifikationsapparaturen können alle technisch üblichen Werkstoffe eingesetzt werden, die im Temperaturbereich bis etwa 250°C gegenüber den zu trennenden Substanzen hinreichend stabil sind. Geeignet sind z.B. Glas, Titan und hochlegierte Edelstähle mit Chrom- und/oder Nickelgehalten von jeweils über 10 Gew.-%, z.B. Werkstoffe nach DIN 1.4571 oder DIN 1.4439.

Eine vorteilhafte Durchführungsform der erfindungsgemässen destillativen Aufarbeitung ist im folgenden dargestellt:

Das einzusetzende Gemisch wird durch Umsetzung von Phenol mit einer Lösung von Peressigsäure oder Perpropionsäure in einem niedriger als Essigsäure siedenden Lösungsmittel wie Benzol, Essigsäureethylester oder 1,2-Dichlorpropan bei einem anfänglichen Molverhältnis von 8 bis 25 mol Phenol pro Mol Percarbonsäure erhalten. Das einzusetzende Gemisch enthält dabei: 8 bis 15 Gew.-Teile niedriger als Essigsäure siedendes Lösungsmittel; 0,5 bis 2 Gew.-Teile Wasser; 3 bis 8 Gew.-Teile Essig- oder Propionsäure; 50 bis 90 Gew.-Teile Phenol; 1,5 bis 4 Gew.-Teile Brenzcatechin; 0,6 bis 3 Gew.-Teile Hydrochinon, und 0,2 bis 2 Gew.-Teile höher als Hydrochinon siedender bzw. nicht destillierbarer Bestandteile, die 2 bis 4 Gew.-% anorganische Salze enthalten und die hauptsächlich aus Kohlenstoff, Sauerstoff und Wasserstoff aufgebaut sind und diese Elemente etwa in folgendem Verhältnis enthalten: 60 bis 80 Gew.-Teile Kohlenstoff, 15 bis 25 Gew.-Teile Sauerstoff und 2 bis 8 Gew.-Teile Wasserstoff.

Dieses Gemisch wird einer Füllkörper- oder Siebbodenrektifikationskolonne aus Glas oder Edelstahl mit 15 bis 20 Trennstufen im Verstärkungs- und bis zu 20 Trennstufen im Abtriebsteil zwischen Abtriebs- und Verstärkungsteil zugeführt.

Die Kolonne wird bei einem Druck zwischen 0,4 und 1,2 bar betrieben, das Verhältnis von Rücklauf zu Entnahme liegt zwischen 0,2:1 und 2:1. Das am Sumpf der Kolonne abgezogene Produkt enthält 40 bis 65 Gew.-% Phenol und praktisch alle höher als Phenol siedenden Bestandteile des in die Kolonne eingesetzten Gemisches und nicht mehr als 3 Gew.-% niedriger als Phenol siedende Anteile. Aus dem Sumpfprodukt werden Brenzcatechin und Hydrochinon durch weitere Rektifikationen, z.B. entsprechend dem in der deutschen Patentanmeldung P Nr. 2928553.8 beschriebenen Verfahren, isoliert. Das Kopfprodukt der ersten Rektifikationskolonne enthält die Hauptmenge des in die Kolonne eingesetzten Phenols sowie den weitaus überwiegenden Teil des Lösungsmittels und der Essig- oder Propionsäure.

Das Kopfprodukt der ersten Rektifikationskolonne wird einer zweiten Rektifikationskolonne aus Glas oder Edelstahl zugeführt. Diese Kolonne weist 15 bis 25 Trennstufen im Verstärkungs- und 15 bis 25 Trennstufen im Abtriebsteil auf. Im Bereich der 5. bis 7. Trennstufe des Abtriebsteils, vom Sumpf aus gerechnet, befindet sich eine Abnahmestelle für einen flüssigen Seitenstrom. Die Kolonne wird unter einem Druck von 0,1 bis 1 bar betrieben, das Verhältnis von Rücklauf zu Entnahme liegt zwischen 0,8:1 und 5:1.

Lösungsmittel, Wasser und Essig- und/oder Propionsäure werden im Gemisch als Kopfprodukt abgezogen. Der Phenolgehalt in diesem Gemisch liegt unter 1 Gew.-%. Aus dem Abtriebsteil entnimmt man flüssiges Phenol. Die Gesamtabnahme ergibt sich aus einer Seitenstromabnahme an Phenol mit bis zu 3 Gew.-% Essig- oder Propionsäure und einer Sumpfabnahme an Phenol mit weniger als 0,1 Gew.-% Essig- oder Propionsäure. Das Mengenverhältnis von Seitenstrom- zu Sumpfentnahme liegt zwischen 80:20 und 99,9:0,1.

Das am Sumpf abgenommene Produkt wird entweder der Umsetzung von Phenol mit der Lösung von Peressig- oder Perpropionsäure zugeführt oder dient als zusätzlicher Rücklauf für die erste Rektifikationskolonne. Das als Seitenstrom entnommene Phenol wird ausschliesslich der Umsetzung von Phenol mit der Percarbonsäure zugeführt. Falls erforderlich, wird das in die Reaktion zurückzuführende Phenol ganz oder teilweise vor der Reaktion einer weiteren Behandlung unterzogen, z.B. mit Ionenaustauschern, wie es in der deutschen Patentanmeldung P Nr. 2928743.2 beschrieben ist.

Das als Kopfprodukt der zweiten Rektifikationskolonne entnommene Gemisch, das Lösungsmittel, Wasser, Essig- oder Propionsäure und nicht mehr als 1 Gew.-% Phenol enthält, wird weiter rektifiziert, um Lösungsmittel und Essig- oder Propionsäure als Reinsubstanzen zu gewinnen und erneut zur Herstellung einer Lösung von Peressig- oder Perpropionsäure zu verwenden. Der nach Abtrennung von Lösungsmittel und Essig- oder Propionsäure vorliegende, die Spuren von Phenol enthaltende Sumpf dieser nachfolgenden Rektifikation wird der ersten oder zweiten Kolonne der erfindungsgemässen destillativen Aufarbeitung als zusätzlicher Zulauf zugeführt.

Gegenüber bekannten Verfahren zur Aufarbeitung ähnlicher Gemische ist beim erfindungsgemässen Verfahren der Aufwand für die Abtrennung und Rückgewinnung von Phenol und niedriger als Phenol siedender Bestandteile verringert. Die erfindungsgemässe destillative Aufarbeitung hat darüber hinaus den Vorteil, dass die im Verfahren auftretenden Hochsieder, die im allgemeinen hochsiedende Oxidationsprodukte des Phenols sind, gelöst bleiben und weder in den Verdampfern, Sümpfen noch anderswo in der Kolonne ausfallen, so dass Verstopfungen vermieden und Ablagerungen auf ein sehr geringes Mass beschränkt bleiben.

Dies ist insbesondere der Fall, wenn Hochsieder und/oder nicht destillierbare Substanzen enthaltende Gemische in den Sumpfbereich der ersten Rektifikationskolonne eingespeist werden.

Es ist überraschend, dass mit einem Verfahren, das einfacher ist als bekannte Verfahren, ein Phenol erhalten werden kann, das ohne weitere aufwendige Massnahmen erneut in die Umsetzung mit Percarbonsäure eingesetzt werden kann. Der Fachmann hätte in Kenntnis der bekannten Verfahren vielmehr erwartet, dass eine verbesserte Durchführung des Verfahrens nur mit einem erhöhten Aufwand möglich sein würde.

*Beispiel*

Das folgende Beispiel soll die Erfindung näher erläutern, ohne sie irgendwie einzuschränken.

12,295 kg/h eines Gemisches, das nach Umsetzung von Phenol mit einer Lösung von Perpropionsäure in einem Gemisch aus Benzol und Propionsäure und nach Abtrennung einer Gasphase nach der Reaktion erhalten wurde, wurde kontinuierlich einer Rektifikationskolonne zwischen Sumpf und Kolonnenkörper zugeführt.

Im zugeführten Gemisch wurden folgende Komponenten bestimmt:

| Benzol | 10,67 Gew.-% |
|---|---|
| Propionsäure | 5,28 Gew.-% |
| Wasser | 0,12 Gew.-% |
| Phenol | 80,02 Gew.-% |
| Brenzcatechin | 2,14 Gew.-% |
| Hydrochinon | 1,38 Gew.-% |

Der nicht bestimmte Rest von 0,39 Gew.-% besteht aus verschiedenen Substanzen. Neben Spuren an niedriger als Phenol siedenden Bestandteilen sind darin nur noch höher als Hydrochinon siedende und nicht destillierbare Substanzen enthalten.

Die Rektifikationskolonne war aus Edelstahl V4A (Werkstoff entsprechend DIN 1.4571) gefertigt und hatte eine wirksame Länge von 5200 mm.

Sie enthielt in ihrem Innern von unten nach oben:
— auf 2000 mm Länge
   8×8 mm Glas-Raschigringe
— auf 1200 mm Länge
   4 Siebböden
— auf 2000 mm Länge
   8×8 mm Glas-Raschigringe

Der innere Kolonnendurchmesser betrug 100 mm.

Vor Eintritt in die Kolonne wurde das Gemisch in einem röhrenförmigen Wärmeaustauscher aus Edelstahl V4A, der mit 6 bar Dampf beheizt wurde, auf eine Produkttemperatur von 142°C aufgeheizt.

Die Kolonne wurde unter einem Druck von 760 mbar, gemessen am Kopf der Kolonne, gehalten. Am Sumpf der Kolonne befand sich ein ebenfalls aus Edelstahl V4A gefertigter Verdampfer, der als Ringspaltverdampfer gebaut war.

Der Verdampfer wurde mit 230°C heissem Wärmeträger-Öl beheizt und die Durchflussmenge an Wärmeträger-Öl so geregelt, dass ein konstantes Temperaturprofil in der Kolonne gehalten wurde. Im Sumpf wurden Temperaturen zwischen 185 und 189°C gemessen, die Temperatur am Kopf der Kolonne betrug 163°C.

Pro Stunde fiel am Sumpf eine Menge von 1,041 kg Produkt und am Kopf eine Menge von 18,173 kg Produkt an.

Das Sumpfprodukt wurde entnommen und in weiteren Rektifikationen entsprechend dem in der deutschen Patentanmeldung P Nr. 2928553.8 beschriebenen Verfahren, auf Brenzcatechin und Hydrochinon aufgearbeitet. Ein bei dieser Aufarbeitung anfallender, überwiegend aus Phenol bestehender Produktstrom von 0,563 kg/h, in dem folgende Bestandteile analysiert wurden:

| | |
|---|---|
| Benzol | 0,04 Gew.-% |
| Brenzcatechin | 0,08 Gew.-% |
| Propionsäure | 2,03 Gew.-% |
| Wasser | 1,20 Gew.-% |
| Phenylpropionat | 0,05 Gew.-% |
| Phenol | 96,60 Gew.-% |

wurde der 1. Rektifikationskolonne 1000 mm unterhalb des Kopfes der Kolonne als zusätzlicher flüssiger Rücklauf zugeführt.

Ferner wurde der 1. Rektifikationskolonne in Höhe des Zulaufs der Hauptmenge zwischen Sumpf und eigentlicher Kolonne noch ein weiterer Produktstrom von 0,066 kg/h, der als Sumpfprodukt der 2. Rektifikationskolonne entnommen wurde, zugeführt. In diesem Produktstrom wurden analysiert:

| | |
|---|---|
| Phenol | 99,54 Gew.-% |
| Propionsäure | 0,04 Gew.-% |
| Wasser | 0,01 Gew.-% |
| Phenylpropionat | 0,41 Gew.-% |

Am Kopf der 1. Rektifikationskolonne fiel eine Menge von 18,173 kg/h Produkt an, in dem folgende Bestandteile analytisch bestimmt wurden:

| | |
|---|---|
| Phenol | 83,38 Gew.-% |
| Propionsäure | 5,37 Gew.-% |
| Benzol | 11,04 Gew.-% |
| Wasser | 0,22 Gew.-% |

Davon wurden 6,295 kg/h kondensiert als flüssiger Rücklauf auf den Kopf der Kolonne zurückgegeben. Die entnommene Menge von 11,878 kg/h wurde kontinuierlich einer zweiten Rektifikationskolonne zwischen Abtriebs- und Verstärkerteil zugeführt.

Diese 2. Rektifikationskolonne war aus Edelstahl V4A gefertigt und hatte einen Innendurchmesser von 100 mm. Im Verstärkerteil war die Kolonne auf 2000 mm Länge mit 8×8 mm Glas-Raschigringen gefüllt. Der Abtriebsteil enthielt vom Zulauf aus betrachtet auf 2500 mm Länge 8×8 mm Glas-Raschigringe, dann in einem Kolonnenstück von 500 mm Länge lediglich eine Vorrichtung zur Entnahme eines flüssigen Seitenstromes (sog. „Entnahmetasse") und daran anschliessend auf 500 mm Länge 8×8 mm Glas-Raschigringe.

Der ebenfalls aus Edelstahl gefertigte Verdampfer der Kolonne hatte einen gewendelten Wärmeaustauscher mit Naturumlauf und wurde mit 10 bar Dampf beheizt. Die Regelung erfolgte durch Änderung des Dampfdruckes zur Haltung eines konstanten Temperaturprofils in der Kolonne.

Am Kopf fiel eine Menge von 8,821 kg/h an, in der folgende Komponenten analysiert wurden:

| | |
|---|---|
| Benzol | 66,87 Gew.-% |
| Propionsäure | 32,04 Gew.-% |
| Phenol | 0,01 Gew.-% |
| Wasser | 1,09 Gew.-% |

Nach der Kondensation wurden 6,861 kg/h als flüssiger Rücklauf auf den Kopf der Kolonne zurückgegeben. Der restliche Kopfanfall wurde entnommen und in zwei weiteren Kolonnen auf Benzol und Propionsäure aufgearbeitet. Das so erhaltene Benzol und die so erhaltene Propionsäure wurden zur Herstellung von Lösungen von Perpropionsäure in einem Gemisch aus Benzol und Propionsäure verwendet.

Der nach Abtrennung von Propionsäure und allen niedriger als diese siedenden Komponenten verbleibende Rest von 0,004 kg/h wurde erneut in die 2. Rektifikationskolonne eingesetzt.

Über die entsprechende Vorrichtung wurde ein flüssiger Seitenstrom von 9,855 kg/h entnommen, in dem folgende Bestandteile analysiert wurden:

| | |
|---|---|
| Phenol | 99,82 Gew.-% |
| Propionsäure | 0,13 Gew.-% |
| Wasser | 0,05 Gew.-% |

Dieser Seitenstrom wurde der Umsetzung mit Perpropionsäurelösung erneut zugeführt.

Aus dem Sumpf der 2. Rektifikationskolonne wurde eine Menge von 0,066 kg/h entnommen.

Dieses Sumpfprodukt wurde der 1. Rektifikationskolonne in gleicher Höhe wie das in die erfindungsgemässe destillative Aufarbeitung einzusetzende Gemisch zugeführt.

## Patentansprüche

1. Verfahren zur Herstellung von Brenzcatechin und Hydrochinon durch Umsetzung von Phenol mit Percarbonsäuren mit 1 bis 4 Kohlenstoffatomen bei einem Molverhältnis Phenol zur Percarbonsäure vor der Reaktion von 5:1 bis 50:1 und Aufarbeitung des nach der Reaktion und ggf. nach weiterer Behandlung vorliegenden Gemisches, das nicht umgesetztes Phenol, die der Percarbonsäure entsprechende Carbonsäure, Brenzcatechin, Hydrochinon und ggf. weitere Bestandteile enthält, unter Verwendung kontinuierlich betriebener Rektifikationsapparate, dadurch gekennzeichnet, dass man

a) das Gemisch kontinuierlich einer ersten Rektifikationskolonne, die bis zu 20 Trennstufen im Abtriebs- und 5 bis 30 Trennstufen im Verstärkungsteil aufweist, zwischen Abtriebs- und Verstärkungsteil oder dem Sumpfbereich zuführt, die Kolonne bei einem Druck zwischen 0,01 und 2 bar betreibt, zwischen 20 und 95 Gew.-% des Kopfproduktes oder die gleiche Gewichtsmenge an Phenol oder an einem Phenol enthaltenden Produktstrom aus dem Verfahren oder die gleiche Gewichtsmenge eines Gemisches aus Kopfprodukt und Phenol und/oder einem Phenol enthaltenden Produktstrom aus dem Verfahren kondensiert als flüssigen Rücklauf oben auf die Kolonne zurückführt, ein Kopfprodukt abzieht, das Phenol und Carbonsäure und ggf. weitere Bestandteile, die tiefer sieden als Phenol, enthält, und ein Sumpfprodukt entnimmt, das Brenzcatechin, Hydrochinon, 40 bis 65 Gew.-% Phenol und ggf. weitere Bestandteile enthält, und aus dem Sumpfprodukt Brenzcatechin und Hydrochinon gewinnt, und

b) das Kopfprodukt der ersten Rektifikationskolonne kontinuierlich einer zweiten Rektifikationskolonne, die 5 bis 35 Trennstufen im Verstärkungs- und 8 bis 35 Trennstufen im Abtriebsteil aufweist, zwischen Abtriebs- und Verstärkungsteil zuführt, diese Kolonne bei einem Druck zwischen 0,02 und 2 bar betreibt, 20 bis 95 Gew.-% des Kopfproduktanfalles kondensiert als flüssigen Rücklauf oben auf die Kolonne zurückführt, ein Kopfprodukt abzieht, das praktisch phenolfrei ist und das die der Percarbonsäure entsprechende Carbonsäure enthält, und dem Abtriebsteil und/oder dem Sumpf der Kolonne ein praktisch reines Phenol entnimmt.

2. Verfahren nach Anspruch 1, dadurch gekennzeichnet, dass ein Gemisch eingesetzt wird, das durch Umsetzung von Phenol mit Peressigsäure oder Perpropionsäure erhalten wurde.

3. Verfahren nach Anspruch 2, dadurch gekennzeichnet, dass als Lösungsmittel für die Percarbonsäure, Wasser, Benzol, Chlorbenzol, 1,2-Dichlorpropan, 1,2-Dichlorethan, Essigsäureethylester, Essigsäure und/oder Propionsäure eingesetzt wurde.

4. Verfahren nach Anspruch 1, dadurch gekennzeichnet, dass Gemische eingesetzt werden, die erhalten wurden durch Umsetzung von Phenol und Percarbonsäure im Molverhältnis von Phenol zu Percarbonsäure zwischen 8 und 25:1.

5. Verfahren nach den Ansprüchen 1 bis 4, dadurch gekennzeichnet, dass produktseitig stets Temperaturen unterhalb von 230°C eingehalten werden.

6. Verfahren nach den Ansprüchen 1 bis 5, dadurch gekennzeichnet, dass man als Rücklauf auf den Kopf der 1. Rektifikationskolonne einen flüssigen Produktstrom verwendet, der dem Abtriebsteil oder dem Sumpf der 2. Rektifikationskolonne entnommen wird.

7. Verfahren nach den Ansprüchen 1 bis 6, dadurch gekennzeichnet, dass man die Brüden der 1. Rektifikationskolonne zur Beheizung der 2. Rektifikationskolonne verwendet.

8. Verfahren nach den Ansprüchen 1 bis 7, dadurch gekennzeichnet, dass man dem Abtriebsteil der 2. Rektifikationskolonne einen Seitenstrom entnimmt und in die Umsetzung mit Percarbonsäure zurückführt.

## Claims

1. Process for the preparation of pyrocatechol and hydroquinone by reaction of phenol with percarboxylic acids with 1 to 4 carbon atoms, with a molar ratio of phenol to percarboxylic acid, before the reaction, of from 5:1 to 50:1, and working-up of the mixture which is present after the reaction and, if appropriate, after further treatment, and which contains unreacted phenol, the carboxylic acid corresponding to the percarboxylic acid, pyrocatechol, hydroquinone and, if appropriate, further constituents, using continuously operated rectification apparatuses, characterised in that

(a) the mixture is continuously fed to a first rectification column between the stripping section and the rectifying section or to the sump section, the rectification column having up to 20 separation stages in the stripping section and 5 to 30 separation stages in the rectifying section, this column is operated under a pressure between 0.01 and 2 bar, between 20 and 95% by weight of the top product or the same quantity by weight of phenol, or of a product stream, containing phenol, from the process, or the same quantity by weight of a mixture of top product and phenol and/or a product stream, containing phenol, from the process, condensed as a liquid reflux, is recycled to the top of the column, a top product, which contains phenol and carboxylic acid and, if appropriate, further constituents, which are lower-boiling than phenol, is withdrawn, and a bottom product is taken off, which contains pyrocatechol, hydroquinone, 40 to 65% by weight of phenol and, if appropriate, further constituents, and pyrocatechol and hydroquinone are recovered from the bottom product, and

(b) the top product of the first rectification column is continuously fed to a second rectification column, between the stripping section and the rectifying section, the second rectification column having 5 to 35 separation stages in the rectifying section and 8 to 35 separation stages in the stripping section, this column is operated under a pressure between 0.02 and 2 bar, 20 to 95% by weight of the product collecting at the head, condensed as a liquid reflux, is recycled to the top of the column, a top product, which is practically free of phenol and which contains the carboxylic acid corresponding to the percarboxylic acid, is withdrawn, and a practically pure phenol is taken off from the stripping section and/or the bottom of the column.

2. Process according to Claim 1, characterised in that the mixture employed has been obtained by reaction of phenol with peracetic acid or perpropionic acid.

3. Process according to Claim 2, characterised in that water, benzene, chlorobenzene, 1,2-dichloropropane, 1,2-dichloroethane, ethyl acetate, acetic acid and/or propionic acid were employed as solvents for the percarboxylic acid.

4. Process according to Claim 1, characterised in that the mixtures employed have been obtained by reaction of phenol and percarboxylic acid in the molar ratio of phenol to percarboxylic acid of between 8:1 and 25:1.

5. Process according to Claims 1 to 4, characterised in that temperatures of below 230°C are always maintained at the product end.

6. Process according to Claims 1 to 5, characterised in that a liquid product stream, which is taken off from the stripping section or the bottom of the second rectification column, is used as a reflux to the head of the first rectification column.

7. Process according to Claims 1 to 6, characterised in that the vapours of the first rectification column are used for heating the second rectification column.

8. Process according to Claims 1 to 7, characterised in that a side stream is taken off from the stripping section of the second rectification column, and is fed back into the reaction with percarboxylic acid.

**Revendications**

1. Procédé de fabrication de pyrocatéchine et d'hydroquinone par réaction du phénol avec des acides percarboxyliques ayant 1 à 4 atomes de carbone, à un rapport molaire du phénol à l'acide percarboxylique avant la réaction de 5:1 à 50:1, et par traitement du mélange se présentant après la réaction et éventuellement après un traitement supplémentaire, lequel contient du phénol n'ayant pas réagi, de l'acide carboxylique correspondant à l'acide percarboxylique, de la pyrocatéchine, de l'hydroquinone et éventuellement d'autres constituants, avec utilisation d'appareils de rectification à fonctionnement continu, caractérisé en ce que

a) on alimente le mélange continuellement dans une première colonne de rectification, qui présente jusqu'à 20 stades de séparation dans la partie d'extraction et 5 à 30 stades de séparation dans la partie de concentration, entre la partie d'extraction et la partie de concentration ou dans la région du fond, on fait fonctionner la colonne sous une pression entre 0,01 et 2 bar, on renvoie en haut de la colonne entre 20 et 95% en poids du produit de tête ou la même quantité en poids de phénol ou d'un courant de produit contenant du phénol et provenant du procédé, ou la même quantité en poids d'un mélange de produit de tête et de phénol et/ou d'un courant de produit contenant du phénol provenant du procédé, à l'état condensé sous forme de reflux liquide, on prélève un produit de tête qui contient du phénol et de l'acide carboxylique, de même qu'éventuellement d'autres constituants bouillant plus bas que le phénol, on retire un produit de fond qui contient la pyrocatéchine, l'hydroquinone, 40 à 65% en poids de phénol et éventuellement d'autres constituants, et l'on récupère à partir du produit de fond la pyrocatéchine et l'hydroquinone,

b) on alimente le produit de tête de la première colonne de rectification continuellement dans une deuxième colonne de rectification, qui présente 5 à 35 stades de séparation dans la partie de concentration et 8 à 35 stades de séparation dans la partie d'extraction, entre la partie d'extraction et la partie de concentration, on fait fonctionner cette colonne sous une pression entre 0,02 et 2 bar, on renvoie 20 à 95% en poids du produit recueilli en tête à l'état condensé comme reflux liquide en haut de la colonne, on prélève un produit de tête qui est pratiquement exempt de phénol et qui contient l'acide carboxylique correspondant à l'acide percarboxylique, et l'on retire de la partie d'extraction et/ou du fond de la colonne un phénol pratiquement pur.

2. Procédé selon la revendication 1, caractérisé en ce qu'on utilise un mélange qui a été obtenu par réaction du phénol avec de l'acide peracétique ou de l'acide perpropionique.

3. Procédé selon la revendication 2, caractérisé en ce qu'on utilise comme solvant pour l'acide percarboxylique de l'eau, du benzène, du chlorobenzène, du 1,2-dichloropropane, du 1,2-dichloréthane, de l'acétate d'éthyle, de l'acide acétique et/ou de l'acide propionique.

4. Procédé selon la revendication 1, caractérisé en ce qu'on utilise des mélanges qui ont été obtenus par réaction du phénol et de l'acide percarboxylique dans le rapport molaire du phénol à l'acide percarboxylique entre 8:1 et 25:1.

5. Procédé selon les revendications 1 à 4, caractérisé en ce que, du côté des produits, on maintient constamment des températures inférieures à 230°C.

6. Procédé selon les revendications 1 à 5, caractérisé en ce qu'on utilise comme reflux en tête de la première colonne de rectification un courant de produit liquide qui a été prélevé dans la partie d'extraction ou dans le fond de la deuxième colonne de rectification.

7. Procédé selon les revendications 1 à 6, caractérisé en ce qu'on utilise les vapeurs de la première colonne de rectification pour le chauffage de la deuxième colonne de rectification.

8. Procédé selon les revendications 1 à 7, caractérisé en ce qu'on prélève à partir de la partie d'extraction de la deuxième colonne de rectification un courant latéral et qu'on le recycle à la réaction avec l'acide percarboxylique.